# FASCICULE DE BREVET EUROPEEN

(11) **EP 0 326 482 B1**
(45) Date de publication et mention de la délivrance du brevet: **09.06.1993**
(21) Numéro de dépôt: 89400198.1
(22) Date de dépôt: 25.01.1989
(51) Int. Cl.: C12P 41/00, C12P 7/40

(54) **Procédé de préparation d'acides aryl-2 alkanoiques optiquement actifs**
Verfahren zur Herstellung von optisch aktiven 2-Arylalkansäuren
Process for the preparation of optically active 2-aryl-alkanoic acids

(30) Priorité: 27.01.1988 FR 8800924
(43) Date de publication de la demande: 02.08.1989
(73) Titulaire: RHONE-POULENC SANTE, 92160 Antony (FR)
(72) Inventeur: Cerbelaud, Edith, F-69005 Lyon (FR); Pétré, Dominique, F-69006 Lyon (FR)
(74) Mandataire: Pilard, Jacques

(56) Documents cités:
- EP-A- 0 227 078
- WO-A-86/07386
- FR-A- 1 342 844
- CHEMICAL ABSTRACTS, vol. 83, no. 3, 21 juillet 1975, page 471, résumé no. 27810q, Columbus, Ohio, US; S. RENDIC et al.: "Resolution of (+/-)-alpha-(#-benzoylphenyl-)propionic acid (Ketoprofen) and diastereometric interaction of its enantiomers with some biological systems"
- CHEMICAL ABSTRACTS, vol. 81, no. 19, 11 novembre 1974, page 376, résumé no. 118551f, Columbus, Ohio, US
- CHEMICAL ABSTRACTS, vol. 91, no. 11, 10 septembre 1979, page 610, résumé no. 89550d, Columbus, Ohio, US

## Description

La présente invention concerne un procédé de préparation d'acides aryl-2 alkanoïques optiquement actifs de formule générale : 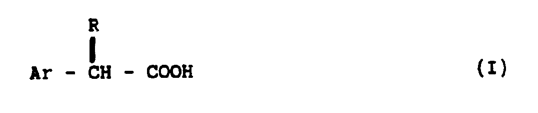 par hydrolyse énantiosélective des amides racémiques correspondants.

Dans la formule générale (I), Ar représente un radical aromatique éventuellement substitué et R représente un radical éthyle, propyle ou isopropyle.

Plus particulièrement, la présente invention concerne la préparation des énantiomères S (+) des acides aryl-2 alkanoïques de formule générale (I).

Plus particulièrement encore, la présente invention concerne la préparation des énantiomères S (+) des acides phényl-2 méthyl-3 butyriques de formule générale : 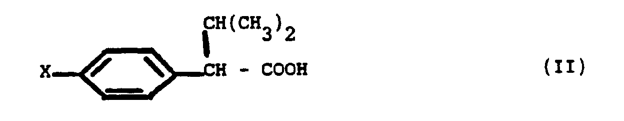
dans laquelle X représente un atome d'halogène, de préférence un atome de chlore, ou un radical méthyle.

Il est connu [Y. Kawakami, J. Synth. Org. Chem., 38, 574 (1980)] que les esters de formule générale : 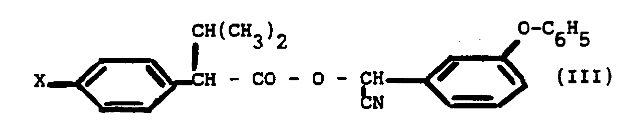 dans laquelle X est défini comme précédemment, possèdent des propriétés insecticides remarquables.

Dans la formule générale (III), la présence de 2 carbones asymétriques conduit à l'existence possible de 4 isomères optiques. Parmi ces isomères, ceux qui dérivent des isomères S (+) des acides de formule générale (II) ont une activité supérieure à celle des produits racémiques correspondants (DE 2 737 297).

Il est connu [H. Nohira et coll., Agric. Biol. Chem., 50, 675 (1986)] de préparer les isomères optiquement actifs des produits de formule générale (II) par cristallisation préférentielle du sel de l'acide de formule générale (II) avec la diéthylamine.

Il a maintenant été trouvé, et c'est ce qui fait l'objet de la présente invention, que les acides aryl-2 alkanoïques de formule générale (I) sous forme S peuvent être obtenus par hydrolyse énantiosélective des amides racémiques correspondants au moyen de microorganismes ou d'enzymes sélectionnés en fonction de leur capacité à hydrolyser l'α-phénylpropionamide racémique en acide α-phénylpropionique S.

La sélection des agents permettant l'hydrolyse énantiosélective des amides des acides aryl-2 alkanoïques de formule générale (I) racémiques est effectuée en mettant en contact l'agent avec l'α-phénylpropionamide en milieu convenable jusqu'à ce que 20% du produit soit converti puis en mesurant l'excès énantiomérique. Sont sélectionnés les agents qui hydrolysent, dans ces conditions, l'α-phénylpropionamide racémique en acide α-phénylpropionique S avec un excès énantiomérique supérieur à 65%.

Parmi les microorganismes qui conviennent particulièrement bien peuvent être citées des souches appartenant au genre Brevibacterium ou Corynebacterium et plus particulièrement Brevibacterium R 312 (CBS 717.73) et Corynebacterium N 771 (FERM P 4445) ou Corynebacterium N 774 (FERM P 4446) qui permettent d'obtenir des acides aryl-2 alkanoïques S avec un excès énantiomérique en isomère S généralement supérieur à 90%.

Il est surprenant de noter que, parmi ces microorganismes, Brevibacterium R 312, qui est décrit dans FR 7 901 803/2 447 359 et dans Advances in Biochemical Engineering, Vol. 14, p. 1-32 (1980) comme possédant une amidase générale non stéréospécifique hydrolyse stéréospécifiquement les amides des acides aryl-2 alkanoïques de formule générale (I) racémiques alors que son mutant A₄ qui possède une amidase L stéréospécifique n'hydrolyse pas les amides des acides aryl-2 alkanoïques de formule générale (I) racémiques.

Il y a lieu de noter que Corynebacterium N 771 et Corynebacterium N 774, qui sont décrits dans EP 0 187 680, hydrolysent par exemple le lactonitrile en acide D,L lactique et l'α-amino-phénylpropionitrile en D,L-phénylalanine sans observer de stéréosélectivité.

Selon la présente invention, le procédé est généralement mis en oeuvre en milieu aqueux ou hydroorganique homogène ou hétérogène, dans des conditions de température et de pH déterminées en fonction de la nature du microorganisme et de l'enzyme, en agitant une suspension de cellules ou d'extraits cellulaires du microorganisme et de l'amide de l'acide aryl-2 alkanoïque racémique.

Le procédé conduit à un mélange de l'acide aryl-2 alkanoïque S et de l'amide de l'acide aryl-2 alkanoïque R, ce dernier pouvant être racémisé selon des techniques connues en amide de l'acide aryl-2 alkanoïque racémique qui peut à nouveau être hydrolysé en acide aryl-2 alkanoïque S dans les conditions données précédemment.

Par exemple la racémisation peut être effectuée par chauffage avec de l'ammoniaque à une température comprise entre 80 et 160°C.

Lorsque le microorganisme utilisé possède la propriété d'hydrolyser les nitriles associée à la propriété d'hydrolyser énantiosélectivement l'α-phénylpropionamide, il est possible d'obtenir un acide aryl-2 alkanoïque S soit à partir du nitrile de l'acide aryl-2 alkanoïque racémique soit à partir de l'amide de l'acide aryl-2 alkanoïque racémique.

Les exemples suivants, donnés à titre non limitatif, montrent comment l'invention peut être mise en pratique.

### EXEMPLE 1

a) La souche Brevibacterium R 312 (CBS 717.73) est cultivée en fiole agitée à 28°C pendant 14 heures dans un milieu dont la composition est la suivante :
- glucose 10 g
- (NH₄)₂ SO₄ 5 g
- KH₂ PO₄ 1,01 g
- Na₂HP₄, 12H₂0 1,64 g
- K₂HPO₄ 0,82 g
- CaCl₂, 2H₂0 0,012 g
- Zn Cl₂ 0,0012 g
- Fe S0₄, 7H₂0 0,0012 g
- MnSO₄, H₂O 0,0012 g
- MgSO₄, 7H₂O 0,5 g
- Chlorhydrate de thiamine 0,002 g
- eau q.s.p. 1000 cm3.

Cette préculture est utilisée pour ensemencer un milieu de culture ayant la même composition mais contenant en plus du N-méthyl-acétamide à la concentration de 20mM. La culture est effectuée en fiole agitée pendant 24 heures à 28°C. La biomasse obtenue est séparée par centrifugation puis elle est lavée deux fois par une solution de chlorure de sodium à 9 g/litre.

b) Un culot de centrifugation contenant 13 mg de cellules de Brevibacterium R 312, exprimés en matières sèches, est mis en suspension dans 2 cm3 de tampon phosphate (50 mM) à pH = 7. On ajoute 25 mg de phényl-2 propionitrile racémique (190 µmoles). Après 24 heures d'agitation à 25°C, le mélange réactionnel est dilué par addition de 23 cm3 d'un mélange acétonitrile-acide chlorhydrique N (90-10 en volumes). Les bactéries sont éliminées par centrifugation. La composition du surnageant est déterminée par chromotographie liquide à haute performance (CLHP).

Le surnageant contient :
119 µmoles de phényl-2 propionamide
62 µmoles d'acide phényl-2 propionique.

On ajoute du chlorure de sodium pour favoriser la séparation des phases aqueuses ou organiques. Après évaporation à sec de la phase organique, le résidu est repris par 20 cm3 d'un mélange chloroforme-soude 0,1N (1-1 en volumes).

La phase basique est acidifiée puis extraite au chloroforme.

La mesure du pouvoir rotatoire montre que l'excès énantiomérique en isomère S (+) est de 100%.

Après dérivation de l'acide phényl-2 propionique avec la R (+) a-méthylbenzylamine, l'analyse par CLHP montre que l'excès énantiomérique est de 96,4%.

### EXEMPLE 2

Un culot de centrifugation contenant 320 mg de cellules de Brevibacterium R 312, exprimés en matières sèches, est mis en suspension dans 5 cm3 de tampon phosphate (50 mM) à pH = 7,0. On ajoute 71,2 mg de (chloro-4 phényl)-2 méthyl-3 butyramide racémique (337 µmoles). Après 117 heures d'agitation à 25°C, le mélange réactionnel est dilué par addition de 45 cm3 d'un mélange acétonitrile-acide chlorhydrique N (90-10 en volumes). Les bactéries sont éliminées par centrifugation. La composition du mélange est déterminée par chromatographie liquide à haute performance (CLHP).

Le surnageant contient :
258 µmoles de (chloro-4 phényl)-2 méthyl-3 butyramide
73 µmoles d'acide (chloro-4 phényl)-2 méthyl-3 butyrique.

On ajoute du chlorure de sodium pour favoriser la séparation des phases aqueuses et organiques. A la phase organique séparée par décantation, on ajoute un mélange éther - soude 0,1 N (3-1 en volumes). Après séparation des phases aqueuses et organiques par décantation et acidification de la phase aqueuse, l'acide (chloro-4 phényl)-2 méthyl-3 butyrique est extrait au chloroforme.

La mesure du pouvoir rotatoire montre que l'excès énantiomérique en isomères S (+) est de 97,5%.

Après dérivation de l'acide avec la R (+) a-méthylbenzyl-amine, l'analyse par CLHP montre que l'excès énantiomérique est supérieur à 90%.

### EXEMPLE 3

Un culot de centrifugation contenant 321 mg de cellules de Brevibacterium R 312, exprimés en matières sèches, est mis en suspension dans 5 cm3 d'un tampon phosphate (50 mM) à pH = 7,0. On ajoute 75,9 mg de (chloro-4 phényl)-2 méthyl-3 butyronitrile racémique (392 µmoles). Après 117 heures d'agitation à 25°C, le mélange réactionnel est dilué par addition de 45 cm3 d'un mélange acétonitrile - acide chlorhydrique N (90-10 en volumes). Les bactéries sont éliminées par centrifugation. La composition du mélange est déterminée par CLHP.

Le surnageant contient :
289 µmoles de (chloro-4 phényl)-2 méthyl-3 butyramide
78 µmoles d'acide (chloro-4 phényl)-2 méthyl-3 butyrique.

L'acide est extrait dans les conditions décrites dans l'exemple 2.

Après dérivation de l'acide avec la R (+) α-méthylbenzyl-amine, l'analyse du mélange par CLHP montre que l'excès énantiomérique de l'acide (chloro-4 phényl)-2 méthyl-3 butyrique est de 97% en isomère S (+).

## Revendications

1. Procédé de préparation des énantiomères S (+) des acides aryl-2 alkanoïques de formule générale : 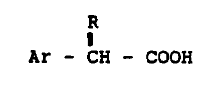 dans laquelle Ar représente un radical aromatique éventuellement substitué et R représente un radical éthyle, propyle ou isopropyle, caractérisé en ce que l'on hydrolyse énantiosélectivement les amides des acides aryl-2 alkanoïques racémiques correspondants, éventuellement préparés in situ, en présence d'un microorganisme ou d'une enzyme issue de ce microorganisme sélectionné en fonction de sa capacité à hydrolyser sélectivement l'α-phénylpropionamide racémique en acide α-phénylpropionique S avec un excès énantiomérique supérieur à 65%, puis sépare l'acide aryl-2 alkanoïque S de l'amide de l'acide aryl-2 alkanoïque R.

2. Procédé selon la revendication 1 caractérisé en ce que les microorganismes sont choisis parmi les Brevibacterium et les Corynebacterium.

3. Procédé selon la revendication 2 caractérisé en ce que les microorganismes sont choisis parmi Brevibacterium R 312 (CBS 717.73), Corynebacterium N 771 (FERM P 4445) et Corynebacterium N 774 (FERM P 4446).

4. Procédé selon la revendication 1 caractérisé en ce que le microorganisme possède une activité nitrilase associée à la capacité d'hydrolyser énantiosélectivement l'α-phénylpropionamide racémique en acide α-phénylpropionique S.

5. Procédé selon la revendication 4 caractérisé en ce que l'on hydrolyse directement le nitrile de l'acide aryl-2 alkanoïque racémique en acide aryl-2 alkanoïque S.

6. Procédé selon l'une des revendications 4 ou 5 caractérisé en ce que les microorganismes sont choisis parmi les Brevibacterium et les Corynebacterium.

7. Procédé selon la revendication 6 caractérisé en ce que les microorganismes sont choisis parmi Brevibacterium R 312 (CBS 717.73), Corynebacterium N 771 (FERM P 4445) et Corynebacterium N 774 (FERM P 4446).

8. Procédé selon l'une des revendications 1 à 7 pour la préparation des acides de formule générale : 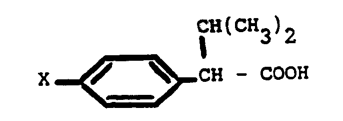 dans laquelle X représente un atome d'halogène ou le radical méthyle, sous forme S (+).

## Claims

1. Process for preparing the S-(+) enantiomers of 2-arylalkanoic acids of general formula: 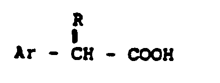 in which Ar represents an optionally substituted aromatic radical and R represents an ethyl, propyl or isopropyl radical, characterised in that the amides of the corresponding racemic 2-arylalkanoic acids, optionally prepared in situ, are enantioselectively hydrolysed in the presence of a microorganism or an enzyme derived from this microorganism which is selected on the basis of its capacity to selectively hydrolyse racemic α-phenylpropionamide to (S)-α-phenylpropionic acid with an enantiomeric excess greater than 65%, and then the (S)-2-arylalkanoic acid is separated from the (R)-2-arylalkanoic acid amide.

2. Process according to Claim 1, characterised in that the microorganisms are chosen from Brevibacterium and Corynebacterium.

3. Process according to Claim 2, characterised in that the microorganisms are chosen from Brevibacterium R 312 (CBS.717.73), Corynebacterium N 771 (FERM P 4445) and Corynebacterium N 774 (FERM P 4446).

4. Process according to Claim 1, characterised in that the microorganism has a nitrilase activity combined with the capacity to enantioselectively hydrolyse racemic α-phenylpropionamide to (S)-α-phenylpropionic acid.

5. Process according to Claim 4, characterised in that the nitrile of the racemic 2-arylalkanoic acid is directly hydrolysed to (S)-2-arylalkanoic acid.

6. Process according to either of Claims 4 and 5, characterised in that the microorganisms are chosen from Brevibacterium and Corynebacterium.

7. Process according to Claim 6, characterised in that the microorganisms are chosen from Brevibacterium R 312 (CBS 717.73), Corynebacterium N 771 (FERM P 4445) and Corynebacterium N 774 (FERM P 4446).

8. Process according to one of Claims 1 to 7, for preparing the acids of general formula: 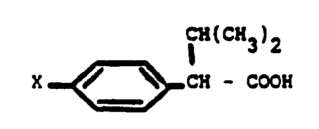 in which X represents a halogen atom or a methyl radical, in S-(+) form.

## Patentansprüche

1. Verfahren zur Herstellung von S(+)-Enantiomeren von 2-Arylalkansäuren der allgemeinen Formel 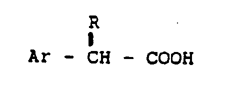 worin Ar einen gegebenenfalls substituierten aromatischen Rest bedeutet, und R für einen Ethyl-, Propyl- oder Isopropylrest steht, dadurch gekennzeichnet, daß man enantioselektiv die Amide der entsprechenden racemischen 2-Arylalkansäuren, die gegebenenfalls in situ hergestellt worden sind, in Anwesenheit eines Mikroorganismus oder eines Enzyms, entstammend diesem Mikroorganismus, ausgewählt in Abhängigkeit von seiner Befähigung, selektiv racemisches α-Phenylpropionamid zu der S-α-Phenylpropionsäure mit einem Enantiomeren-Überschuß von mehr als 65 % zu hydrolysieren, hydrolysiert, wonach man die S-2-Arylalkansäure von dem Amid der R-2-Arylalkansäure abtrennt.

2. Verfahren gemäß Anspruch 1, dadurch gekennzeichnet, daß die Mikroorganismen unter Brevibacterium und Corynebacterium ausgewählt werden.

3. Verfahren gemäß Anspruch 2, dadurch gekennzeichnet, daß die Mikroorganismen unter Brevibacterium R 312 (CBS 717.73), Corynebacterium N 771 (FERM P 4445) und Corynebacterium N 774 (FERM P 4446) ausgewählt werden.

4. Verfahren gemäß Anspruch 1, dadurch gekennzeichnet, daß der Mikroorganismus eine Nitrilase-Aktivität besitzt, die mit der Befähigung, enantioselektiv racemisches α-Phenylpropionamid zu der S-α-Phenylpropionsäure zu hydrolysieren, assoziert ist.

5. Verfahren gemäß Anspruch 4, dadurch gekennzeichnet, daß man direkt das Nitril der racemischen 2-Arylalkansäure zu der S-2-Arylalkansäure hydrolysiert.

6. Verfahren gemäß einem der Ansprüche 4 oder 5, dadurch gekennzeichnet, daß die Mikroorganismen unter Brevibacterium und Corynebacterium ausgewählt werden.

7. Verfahren gemäß Anspruch 6, dadurch gekennzeichnet, daß die Mikroorganismen unter Brevibacterium R 312 (CBS 717.73), Corynebacterium N 771 (FERM P 4445) und Corynebacterium N 774 (FERM P 4446) ausgewählt werden.

8. Verfahren gemäß einem der Ansprüche 1 bis 7 zur Herstellung von Säuren der allgemeinen Formel 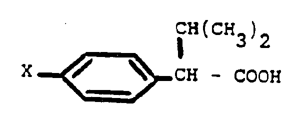 worin X ein Halogenatom oder den Methylrest bedeutet, in der S(+)-Form.
